# EUROPEAN PATENT SPECIFICATION

(11) **EP 2 160 384 B1**
(45) Date of publication and mention of the grant of the patent: **17.04.2013**
(21) Application number: 08750212.6
(22) Date of filing: 08.05.2008
(51) Int. Cl.: C07D 257/04, C07D 413/10

(54) **PROCESS FOR THE PREPARATION OF VALSARTAN**
VERFAHREN ZUR HERSTELLUNG VON VALSARTAN
PROCÉDÉ DE PRÉPARATION DU VALSARTAN

(30) Priority: 14.05.2007 ES 200701365
(43) Date of publication of application: 10.03.2010
(73) Proprietor: Chemo Ibérica, S.A., 08028 Barcelona (ES)
(72) Inventor: COSME GOMEZ, Antonio, E-28813 Torres De Alameda (Madrid) (ES); PALOMO NICOLAU, Francisco Eugenio, E-28804 Alcala De Henares (Madrid) (ES)
(74) Representative: ZBM Patents
(86) International application number: PCT/EP2008/055719
(87) International publication number: WO 2008/138871

(56) References cited:
- EP-A- 1 533 305

## Description

### Field of the invention

The present invention relates to a new process for the preparation of Valsartan.

### Background

Valsartan is the INN of the chemical compound *N*-(1-oxopentyl)-*N*-[[2'-(1*H-*tetrazol-5-yl)[1,1'-biphenyl]-4-yl]methyl]-L-valine corresponding to the structure:

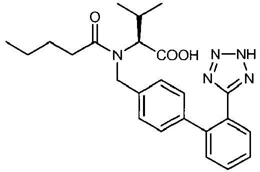

Valsartan is comprised in the group of the antagonists of angiotensin II receptor, which are used in the treatment of hypertension, anxiety, glaucoma, and cardiac stroke. Valsartan is used in medicine as an antihypertensive agent and also in cases of cardiac insufficiency. Valsartan and processes for the preparation thereof were disclosed in the European Patent Application EP-A-443983. In those processes there is the need of using chromatographic techniques to isolate and/or to purify some of the intermediates implied therein.Thus, the complex equipment and processes required for the industrial application of the disclosed processes impair the scalability thereof.

Therefore, there are a number of alternative processes for the preparation of Valsartan in the state of the art which attempt to overcome the disadvantages of the original process. Among them, processes disclosed in the following patent applications can be mentioned: EP-A-1533305, EP-A-1555260, US-A-2006/149079, WO-A-93/10106, WO-A-99/01459, WO-A-2004/026847, WO-A-2004/094391, WO-A-2004/101534, WO-A-2004/111018, WO-A-2005/014602, WO-A-2005/021535, WO-A-2005/049586, WO-A-2005/51928, WO-A-2005/51929, WO-A-2005/102987, WO-A-2006/058701, WO-A-2007/005967, and WO-A-2007/032019.
Depending on the strategy of synthesis used, in some of said processes there is the need of using protecting groups to avoid the reaction of the tetrazole ring in the reaction conditions of some of the steps. For example, the processes disclosed in EP-A-1533305, EP-A-1555260, WO-A-93/10106, WO-A-99/01459, WO-A-2004/094391, WO-A-2005/51928, WO-A-2005/51929, and WO-A-2005/021535 patent applications comprise the use of protecting groups for the tetrazole ring. The introduction and elimination of protecting groups from a compound comprising various functional groups is a common technique in the field of the organic synthesis, and said reactions are typically performed without significant problems. Said procedure is disclosed in well known reference books as, for example, in T. W. Greene et a/., Protective Groups in Organic Synthesis, 3rd Edition, John Wiley & Sons, New York, 1999 [ISBN: 0-471-16019-9].
The process disclosed in the European Patent Application EP-A-1533305 for the preparation of Valsartan is shown is Scheme I:

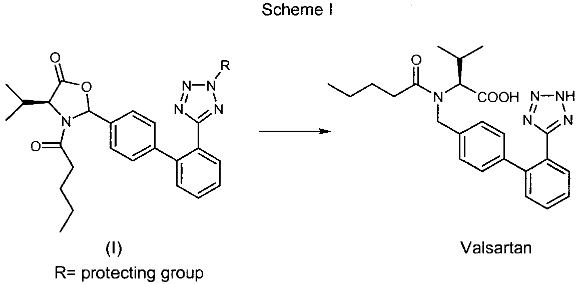

As shown, the process implies the compound of general formula (I) as an intermediate, which contains the tetrazole ring protected by a protecting group R.
According to the description of the patent application, the protecting group can be triphenylmethyl, *t*-butyl, C₁-C₄-alkoxymethyl, methylthiomethyl, phenyl-(C₁-C₄) alkoxymethyl, p-methoxybenzyl, 2,4,6-trimethylbenzyl, 1-methyl-1-phenylethyl, 2-(trimethylsilyl)ethyl, tetrahydropyranyl, piperonyl, or benzenesulfonyl, preferably, the group used is triphenylmethyl (trityl), 1-methyl-1-phenylethyl (cumyl) or *t*-butyl.
The opening of the oxazolidinone ring of the compound of general formula (I) is performed by hydrogenolysis, which can be performed by catalytic hydrogenation or by catalytic hydrogen transfer through a hydrogen donor, such as: formic acid, formiates, ascorbic acid, phosphinic acid, phosphinates, phosphites, and alcohols. The elimination of the protecting agent can also occur during the hydrogenolysis, i.e. immediately before, simultaneously or immediately after the opening of the oxazolidinone ring. The examples disclosed in the European Patent Application EP-A-1533305 show the preparation of Valsartan through the hydrogenolysis of the compound of general formula (I) wherein the protecting group is the trityl group:
- In Example 4, the hydrogenolysis is carried out with ammonium formiate as hydrogen donor in the presence of the 5% Pd/C catalyst, and Valsartan is obtained yielding 70% after a treatment comprising the acidification of the reaction mixture with 20% sulphuric acid.
- In Example 5, the opening of the oxazolidinone ring is carried out with a pressure of hydrogen in the presence of the same palladium catalyst, and Valsartan is obtained yielding 53% after the treatment of Example 4. Therefore, the preparation of Valsartan from the compound of formula (I) shows a deficient yield for the industrial scalability, particularly in the case of catalytic hydrogenation.
The authors of the present invention have observed that the yield loss of said reaction is due to the presence of side reactions leading to the degradation of the compound of general formula (I) in the reaction conditions. Said reactions lead to the formation of impurities from the compound of general formula (I), thus lowering the yield. Generally, the presence of impurities may contribute to making the industrial process more complex because it is more difficult to control, and may be necessary to use additional purification processes to obtain a pharmaceutically acceptable product. Thus, there is the need to have at one's disposal an improved process for the preparation of Valsartan with an improved yield and a suitable purity to be used as active pharmaceutical ingredient.

### Summary of the invention

The authors of the present invention have found a new process for the preparation of Valsartan starting from the compound of general formula (I) wherein the yield is significantly improved and the formation of impurities is reduced. The object of the invention is a process for the preparation of Valsartan starting from the compound of general formula (I).

### Detailed Description of the Invention

The process for the preparation of Valsartan found by the inventors may be depicted by Scheme II:

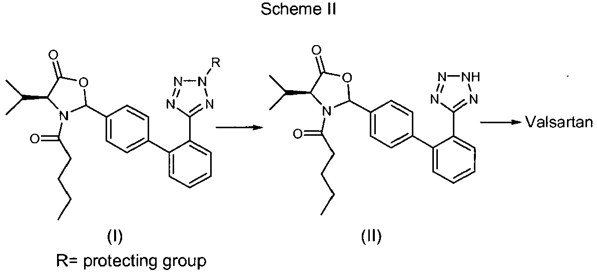

Compound of general formula (I) comprises the tetrazole ring protected by a protecting group. In a first step, said protecting group is eliminated by the treatment with sulphuric acid, and the compound of formula (II) is obtained. Next, the oxazolidinone ring of compound (II) is opened by catalytic hydrogenation and Valsartan, or a pharmaceutically acceptable salt thereof, is obtained. Surprisingly, global yield of both steps is from 80% to 90%, showing that the developed process is advantageous over the process disclosed in patent application EP-A-1533305, which shows significantly lower yields.
The process for the preparation of Valsartan, which is the object of the invention, comprises the reaction of a compound of general formula (I): 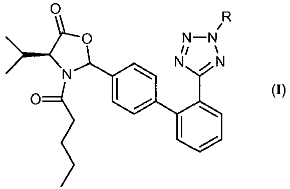 wherein R is a protecting agent selected from the group consisting of 1-methyl-1-phenylethyl, triphenylmethyl and t-butyl, with concentrated sulphuric acid to obtain the compound of formula (II): 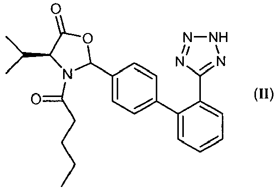 next, a catalytic hydrogenation of compound (II) in the presence of an organic base, and the isolation of Valsartan or a pharmaceutically acceptable salt thereof.

### Starting compound

Starting compound of general formula (I) in the process of the invention may be obtained, for instance, by an analogue process to that disclosed in Example 3 of EP-A-1533305 patent application, through a coupling of:
- the compound obtained in Example 2 of said patent application, and
- a boronic acid derivative obtained according to the process disclosed in WO-A-93/10106 patent application from a compound of general formula (III) 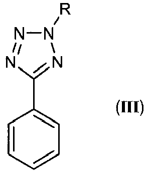 wherein R is 1-methyl-1-phenylethyl, triphenylmethyl, or *t*-butyl.
Compounds of general formula (III) may be prepared, for instance, according to the processes disclosed in WO-A-95/32962 and WO-A-93/10106 patent applications.

The 1-methyl-1-phenylethyl group is generally referred to as cumyl group, and triphenylmethyl is referred to as trityl group. According to the process of the invention, cumyl protective group is preferably used.

### Reaction with concentrated sulphuric acid

The first step of the process of the present invention comprises the elimination of the protecting group of the tetrazole ring being part of the compound of general formula (I).
Sulphuric acid used in the process of the invention is concentrated. As used herein, concentrated sulphuric acid refers to a sulphuric acid in a concentration exceeding 90% w/w, preferably exceeding 95% w/w, and more preferably exceeding 96%. Generally, the reaction of a compound of general formula (I) and the concentrated sulphuric acid is carried out at a temperature below room temperature, preferably below 15° C, and more preferably from 5° C to 10° C.
Generally, treatments with concentrated sulphuric acid may be carried out directly using the product or a solution thereof in an inert solvent. According to the process of the invention, a compound of general formula (I) is generally solved in an inert solvent, e.g. methylene chloride or chlorophorm, to carry out the deprotection of the protective group with sulphuric acid.
The solution of said compound is usually added slowly over the sulphuric acid maintaining the reaction temperature below room temperature, preferably below 15° C, and more preferably from 5° C to 10° C.
Generally, the addition of the compound of general formula (I) over chilled sulphuric acid is carried out in a period of time comprised from 30 minutes to 3 hours. Generally, the temperature of the reaction mixture is maintained from 5° C to 10° C during the whole process. The deprotection reaction is very fast, thus the temperature of the reaction mixture is generally maintained at the temperature mentioned above from 5 minutes to 2 hours, preferably from 15 minutes to 1 hour.
The obtained product, compound of formula (II), may be isolated according to common methods known by the skilled in the art, for instance, by treatment of the reaction mixture with a mixture of water and a solvent, such as isopropylacetate. Generally, compound of formula (II) is obtained pure with a yield close to 90%. The obtained product may be used without further purification in the next step of the process of the invention for the preparation of Valsartan.

### Hydrogenation reaction

The second step of the process of the invention comprises the opening of the oxazolidinone ring of compound of formula (II) by treatment with hydrogen in the presence of a catalyst and an organic base. Catalytic hydrogenation is a well-known reaction for the skilled in the art wherein hydrogen gas and a catalyst are used.
Depending on the catalyst used, hydrogen may be employed at low pressure, from 1 to 4 bars, and at relatively low temperatures, from 0° C to 100° C, or at high pressure, from 100 and 400 bars, and higher temperatures, from 25° C to 300° C. According to the process of the invention, hydrogen is generally used at low pressure, preferably from 1 to 4 bars, and more preferably from 2 and 3 bars. In low-pressure catalytic hydrogenations, a catalyst based in noble metals is generally used, e.g. based on palladium, rhodium, or ruthenium. Hydrogenation reactions over platinum are frequently carried out using a platinum fine powder obtained through the reduction of a platinum compound, for example platinum oxide. Otherwise, palladium, rhodium, and ruthenium catalysts are used in the form of a metal deposition over an inert support as carbon, alumina, barium sulphate, calcium carbonate, or strontium carbonate. The above catalysts are heterogeneous catalysts, among them: Pt/C, Rh/C, Pd/C, Ru/C, Pd/BaSO4, and Rh/alumina. Homogeneous catalysts may also be used, e.g. PdCl₂(PPh₃)₂, o PtCl₂(PPh₃)_{2.}
The catalyst used according to the process of the invention is preferably selected from the group formed by heterogeneous catalysts, preferably Pd/C, and more preferably 10% Pd/C. The hydrogenation reaction is carried out in the presence of an organic base. Among suitable organic bases, those soluble in an organic solvent are preferred, e.g. diisopropylamine, dibutylamine, triethylamine, dicyclohexylamine, and piperidine. Preferably, dicyclohexylamine is used.
Compound of formula (II) is solid and, in order to carry out the hydrogenation reaction, it is generally dissolved in a solvent as toluene, isopropanol, methanol, ethanol, tetrahydrofurane, or mixtures thereof.
The hydrogenation reaction is generally carried out under stirring and the temperature of the reaction is from 20° C to 80° C, preferably from 50° C to 70° C, more preferably from 60° C to 65° C. Hydrogenation reaction may be carried out in a conventional steel hydrogenator suitable for a pressure from 1 to 4 bars. The progress of the reaction is monitored by HPLC. Generally, the reaction is considered complete when the compound of formula (II) remaining in the reaction mixture is less than 1%. Generally, the reaction is completed from 4 to 6 hours after the initiation.
Valsartan may be isolated from the reaction mixture by a conventional treatment, for instance that disclosed in Example 5 of EP-A-1533305 patent application.
Through the process of the invention, Valsartan is generally obtained as a white solid with yields higher than 90%, with a suitable purity for its use as active ingredient in pharmaceutical formulations. From the compound of general formula (I), the global yield of the process of the invention is usually more than 80%.
Valsartan obtained through the process of the invention may be transformed in any of the pharmaceutical suitable salts thereof, as described in WO-A-92/06253 patent application. The process of the invention have shown a noticeably reduced formation of impurities when compared with a process which is an analogue of that disclosed in Example 5 of EP-A-1533305 patent application, wherein compound of general formula (I) is hydrogenated directly obtaining Valsartan.
For instance, when the direct hydrogenation of the compound of general formula (I) wherein R is cumyl is carried out in the same conditions of the second step of the process of the invention, the following additional impurities have been identified, in addition to isopropylbenzene arising from cumyl protective group:

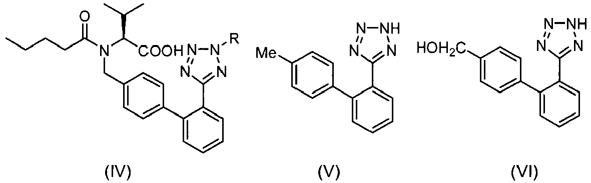

The percentages of the various components of the reaction mixture determined by HPLC at different reaction times are shown in Table I:

**Table I**

| **Compound** | **1.5 h** | **6.5 h** | **11.5 h** | **15.5 h** | **18.5 h** |
|---|---|---|---|---|---|
| (I) wherein R=cumyl | 48.62% | 18.72% | 13.97% | 8.29% | 2.95% |
| **Valsartan** | 15.07% | 35.30% | 41.02% | 54.58% | 71.41% |
| (II) | 25.78% | 28.66% | 26.69% | 17.97% | 6.33% |
| (IV) wherein R=cumyl | 3.01% | 3.49% | 3.10% | 2.48% | 1.24% |
| (V) | 0.11% | 1.04% | 2.70% | 6.51% | 8.82% |
| (VI) | 2.86% | 6.33% | 5.21% | 2.65% | 1.30% |
| Isopropylbenzene | 2.21% | 3.41% | 4.57% | 4.97% | 4.97% |

As shown, compound of general formula (I) wherein R is cumyl reacts slowly, and the impurity content increases with time.
It can be also observed that hydrogenation conditions used in this reaction, which are those of the second step of the process of the invention, lead to the preparation of Valsartan yielding about 70%. This value is noticeably higher than that obtained through the hydrogenation reaction of Example 5 of EP-A-1533305 patent application, which is 53%. When Valsartan is prepared through the hydrogenation of compound of formula (II) according to the process of the invention, the impurity content is noticeably lower, and that increase the yield of said active ingredient.

Table II shows the percentages of the various components of the reaction mixture when reaction is complete after a hydrogenation as described in Example 2:

**Table II**

| **Compound** | **Content** |
|---|---|
| (II) | 0.09% |
| Valsartan | 95.6% |
| (V) | 2.22% |
| (VI) | 0.55% |

In this case, isopropylbenzene is not detected because cumyl group is removed in the first step of the process of the invention by treatment with sulphuric acid. It can be noted that starting compound, compound of formula (II), have disappeared substantially after 4-5 h over hydrogenation, and that the impurity content is noticeably low and clearly lower to that obtained in a direct hydrogenation, as shown in Table I. Therefore, the process of the invention is a new process for the preparation of Valsartan which allows the preparation thereof from compound of general formula (I) with an improved yield and a suitable purity to be used as active ingredient in pharmaceutical compositions. The following examples are provided by way of illustration, and are not intended to be limiting of the present invention.

### Examples

### Example 1.- Preparation of Compound of formula (II)

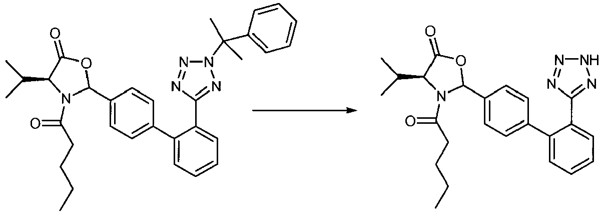

100 ml (0.18 mol) of concentrated sulphuric acid were placed in a 500 ml flask with magnetic stirring and a thermometer and the temperature was set from 0° C to 10° C by an external water-ice bath. Once said temperature was achieved, the addition of 100 g (0.18 mol) of the compound of general formula (I) wherein R is cumyl, e.g. prepared according to the process described in "Starting compound" herein, solved in 167 ml of methylene chloride, was started by an addition funnel, maintaining the temperature from 5° C to 10° C. When the addition was finished, the temperature of the reaction mixture was maintained from 5° C and 10° C until the reaction was completed. The reaction mixture was intense red. Thereafter, the reaction mixture was poured over a mixture of 425 ml of isopropyl acetate and 1000 ml of water through an addition funnel, maintaining the temperature from 5° C to 10° C. Next, the mixture was poured in a separation funnel and the phases were separated. The lower aqueous phase, not containing product, was washed with 112 ml of isopropyl acetate. The obtained organic phases were joined, dried and vacuum-concentrated. 112 ml of isopropylacetate were added to the yellowish solid obtained and the mixture was stirred at room temperature for 30 minutes. Next, the mixture was cooled between -5° C and 0° C, stirring at said temperature for 2 hours. The mixture was filtered and the recovered solid was washed with 80 ml of cold isopropylacetate. 84 g of wet product were obtained and dried under vacuum in a furnace at 40° C. Finally, 68.68 g of pure compound of formula (II) were obtained, thus yielding 87%.

### Example 2.- Preparation of Valsartan from compound of formula (II)

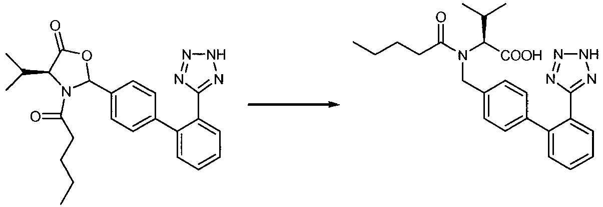

24.3 g of 10% Pd/C and 325 ml of water were placed in a 1 litre steel hydrogenator. Next, 50 g (0.12 mol) of compound of formula (II) obtained in Example 1 were added dissolved in a mixture of 250 ml of isopropanol, 100 ml of toluene and 41.4 ml (0.23 mol) of dicyclohexylamine. The mixture was stirred at 400 rpm and nitrogen was added to provide an inert atmosphere. The system was checked for leaks, and 3 bars of hydrogen pressure were added, stirring was increased to 1000 rpm and heating to 65° C of the recirculation water was started. The progression of the hydrogenation was monitored by HPLC, the reaction was considered completed 5 hours later, when less than 1% of the starting product remained unreacted.
The mixture was extracted from the hydrogenator washing the system with 80 ml of isopropanol. pH was set at 12 by adding about 22 ml of 30% solution of sodium hydroxide. Next, the mixture was filtered with a filtering adjuvant, e.g. Hyflo, and washed with 200 ml of a mixture of isopropanol/water/toluene 3:4:1 v/v. Phases were decanted, and the aqueous phase was washed with 150 ml of toluene.
350 ml of isopropylacetate were added to the alkaline aqueous phase, and pH was set to 2 by the addition of about 19 ml of concentrated hydrochloric acid. The phases were decanted, and the aqueous phase was extracted again with 350 ml of isopropylacetate. The organic phases were joined and vacuum-concentrated. The resulting material was washed with 200 ml of isopropylacetate and concentrated again. 350 ml of isopropylacetate were added to the white solid obtained and the moisture of the mixture was determined to check that it did not exceeded 1%. The mixture was heated at 40° C until dissolution. Next, the mixture was cooled at room temperature for 30 minutes and a white solid started to precipitate. 350 ml of hexane were added to the precipitated and it was cooled from 0° C to 5° C for 1 hour in order to complete the precipitation. The obtained solid was filtered and washed with 70 ml of a cold mixture of isopropyl acetate/heptane 1:1 v/v. The wet solid was dried in a furnace at 40° C under vacuum, and finally 46.65 g of pure Valsartan were obtained, with a yield of 94%.

### Comparative Example 1.- Preparation of Valsartan by direct hydrogenation starting from compound of general formula (I) wherein R is cumyl.

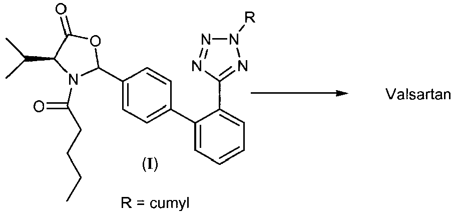

Following a similar process to that described in Example 2, compound of general formula (I) wherein R is cumyl was hydrogenated with hydrogen using Pd/C as catalyst and in the presence of dicyclohexylamine as organic base. Samples of the reaction mass were collected at different times: 1.5 h, 6.5 h, 11.5 h, 15.5, h and 18.5 h, and were analyzed by HPLC to determine the content of the different compounds. The results are shown in Table I in the description. Finally, Valsartan was isolated in 55% yield.

## Claims

1. Process for the preparation of Valsartan **characterized in that** it comprises the reaction of a compound of general formula (I): 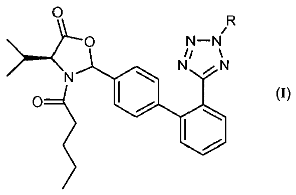 wherein R is a protecting group selected from the group consisting of 1-methyl-1-phenylethyl, triphenylmethyl and *t*-butyl, with concentrated sulphuric acid to obtain a compound of formula (II): 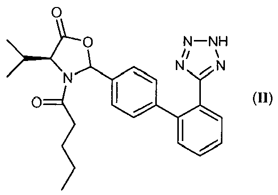 next, a catalytic hydrogenation of compound (II) in the presence of an organic base, and the isolation of Valsartan or a pharmaceutically acceptable salt thereof.

2. Process according to claim 1, **characterized in that** R is cumyl.

3. Process according to any one of the claims 1-2, **characterized in that** the concentration of sulphuric acid is more than 90% w/w.

4. Process according to claim 3, **characterized in that** the concentration of sulphuric acid is more than 95% w/w.

5. Process according to any one of the claims 1-4 **characterized in that** the reaction of the compound of general formula (I) and the concentrated sulphuric acid is carried out at a temperature below room temperature.

6. Process according to claim 5, **characterized in that** the temperature is below 15° C.

7. Process according to claim 6, **characterized in that** the temperature is comprised from 5° C to 10° C.

8. Process according to any one of the claims 1-7 **characterized in that** the hydrogenation reaction is performed at a pressure comprised from 1 to 4 bar.

9. Process according to any one of the claims 1-8 **characterized in that** the catalyst for the hydrogenation reaction is selected from the group consisting of heterogeneous catalysts.

10. Process according to claim 9, **characterized in that** the catalyst is Pd/C.

11. Process according to claim 10, **characterized in that** the catalyst is 10% Pd/C.

12. Process according to any one of the claims 1-11, **characterized in that** the organic base is dicyclohexylamine.

13. Process according to any one of the claims 1-12, **characterized in that** the hydrogenation reaction is performed at a temperature comprised from 20° C to 80° C.

14. Process according to claim 13, **characterized in that** the temperature is comprised from 50° C to 70° C.

15. Process according to claim 14, **characterized in that** the temperature is comprised from 60° C to 65° C.

## Patentansprüche

1. Verfahren zur Herstellung von Valsartan **dadurch gekennzeichnet, dass** es die Reaktion einer Verbindung der allgemeinen Formel (I): 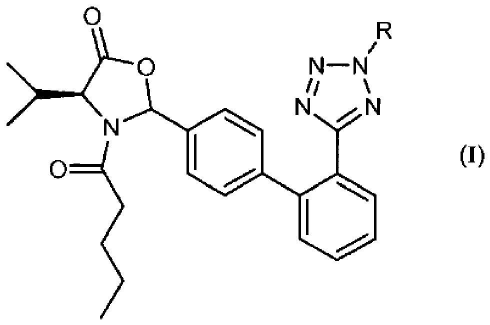 mit konzentrierter Schwefelsäure umfasst, wobei R eine Schutzgruppe ist, die ausgewählt aus der Gruppe bestehend aus 1-Methyl-1-phenylethyl, Triphenyl-methyl und t-Butyl ist, um eine Verbindung der Formel (II): 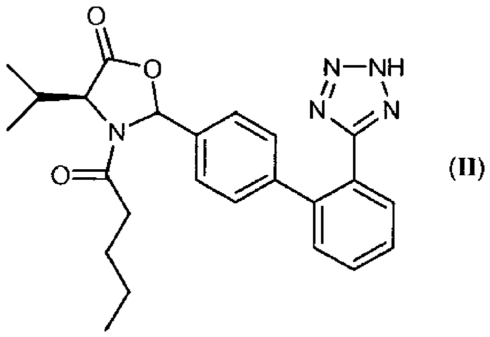 zu gewinnen, gefolgt von katalytischer Hydrierung der Verbindung (II) in Anwesenheit von einer organischen Base, und die Isolierung von Valsartan oder einem pharmazeutisch akzeptablen Salz davon.

2. Verfahren nach Anspruch 1 **dadurch gekennzeichnet, dass** R Cumyl ist.

3. Verfahren nach einem der Ansprüche 1-2 **dadurch gekennzeichnet, dass** die Konzentrierung von Schwefelsäure höher als 90 % Gew/Gew ist.

4. Verfahren nach Anspruch 3 **dadurch gekennzeichnet, dass** die Konzentrierung von Schwefelsäure höher als 95 % Gew/Gew ist.

5. Verfahren nach einem der Ansprüche 1-4 **dadurch gekennzeichnet, dass** die Reaktion der Verbindung der allgemeinen Formel (I) und der konzentrierten Schwefelsäure bei einer Temperatur unterhalb der Raumtemperatur durchgeführt wird.

6. Verfahren nach Anspruch 5 **dadurch gekennzeichnet, dass** die Temperatur bei unter 15 °C liegt.

7. Verfahren nach Anspruch 6 **dadurch gekennzeichnet, dass** die Temperatur von 5 °C bis 10 °C reicht.

8. Verfahren nach einem der Ansprüche 1-7 **dadurch gekennzeichnet, dass** die Hydrierungsreaktion bei einem Druck von 1 bis 4 bar durchgeführt wird.

9. Verfahren nach einem der Ansprüche 1-8 **dadurch gekennzeichnet, dass** der Katalysator für die Hydrierungsreaktion ausgewählt aus der Gruppe bestehend aus heterogenen Katalysatoren ist.

10. Verfahren nach Anspruch 9 **dadurch gekennzeichnet, dass** der Katalysator Pd/C ist.

11. Verfahren nach Anspruch 10 **dadurch gekennzeichnet, dass** der Katalysator 10% Pd/C ist.

12. Verfahren nach einem der Ansprüche 1-11 **dadurch gekennzeichnet, dass** die organische Base Dicyclohexylamin ist.

13. Verfahren nach einem der Ansprüche 1-12 **dadurch gekennzeichnet, dass** die Hydrierungsreaktion bei einer Temperatur von 20 °C bis 80 °C durchgeführt wird.

14. Verfahren nach Anspruch 13 **dadurch gekennzeichnet, dass** die Temperatur von 50 °C bis 70 °C reicht.

15. Verfahren nach Anspruch 14 **dadurch gekennzeichnet, dass** die Temperatur von 60 °C bis 65 °C reicht.

## Revendications

1. Procédé de préparation de Valsartan **caractérisé en ce qu'**il comprend la réaction d'un composé de formule générale (I) : 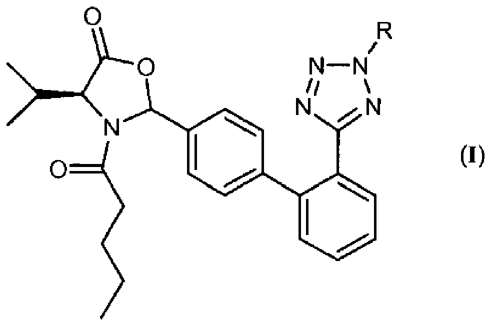 dans laquelle R est un groupe protecteur choisi dans le groupe constitué du 1-méthyl-1-phényléthyle, triphényl-méthyle et t-butyle, avec de l'acide sulfurique concentré afin d'obtenir un composé de formule (II): 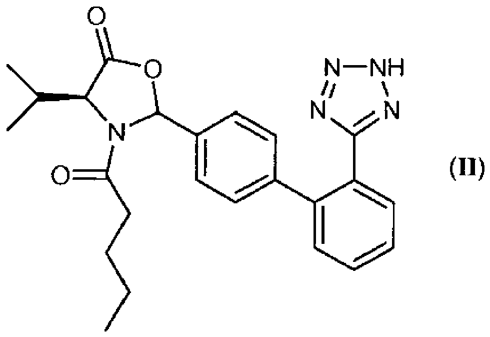 suivie d'une hydrogénation catalytique du composé (II) en présence d'une base organique et l'isolation de Valsartan ou d'un sel pharmaceutiquement acceptable de celui-ci.

2. Procédé selon la revendication 1 **caractérisé en ce que** R est le cumyle.

3. Procédé selon l'une quelconque des revendications 1-2 **caractérisé en ce que** la concentration d'acide sulfurique est supérieure à 90% p/p.

4. Procédé selon la revendication 3 **caractérisé en ce que** la concentration d'acide sulfurique est supérieure à 95% p/p.

5. Procédé selon l'une quelconque des revendications 1-4 **caractérisé en ce que** la réaction du composé de formule générale (I) et de l'acide sulfurique concentré est effectuée à une température inférieure à la température ambiante.

6. Procédé selon la revendication 5 **caractérisé en ce que** la température est inférieure à 15 °C.

7. Procédé selon la revendication 6 **caractérisé en ce que** la température va de 5 °C à 10 °C.

8. Procédé selon l'une quelconque des revendications 1-7 **caractérisé en ce que** la réaction d'hydrogénation est effectuée à une pression de 1 à 4 bar.

9. Procédé selon l'une quelconque des revendications 1-8 **caractérisé en ce que** le catalyseur pour la réaction d'hydrogénation est choisi dans le groupe constitué des catalyseurs hétérogènes.

10. Procédé selon la revendication 9 **caractérisé en ce que** le catalyseur est le Pd/C.

11. Procédé selon la revendication 10 **caractérisé en ce que** le catalyseur est le Pd/C à 10%.

12. Procédé selon l'une quelconque des revendications 1-11 **caractérisé en ce que** la base organique est la dicyclohéxylamine.

13. Procédé selon l'une quelconque des revendications 1-12 **caractérisé en ce que** la réaction d'hydrogénation est effectuée à une température allant de 20 °C à 80 °C.

14. Procédé selon la revendication 13 **caractérisé en ce que** la température va de 50 °C à 70 °C.

15. Procédé selon la revendication 14 **caractérisé en ce que** la température va de 60 °C à 65 °C.
